# EUROPEAN PATENT APPLICATION

(11) **EP 1 640 002 A1**
(43) Date of publication of application: **29.03.2006**
(21) Application number: 05291767.1
(22) Date of filing: 23.08.2005
(51) Int. Cl.: A61K 31/416, A61P 35/00

(54) **Indazole derivatives for inhibition of angiogenesis**

(30) Priority: 30.08.2004 US 930065
(71) Applicant: Yung Shin Pharmaceutical Ind. Co., Ltd., Tachia T'ai chung (TW)
(72) Inventor: Teng, Che-Ming, Da-an District Tapei, 106 (TW); Kuo, heng-Chu, Taiichung (TW); Lee, Fang-Yu, Tachia Taichung (TW); Pan, Shiow-Lin, Da-an District Taipei (TW); Guh, Jih-Hwa, Sin-yi District Taipei, 110 (TW)
(74) Representative: Nargolwalla, Cyra

(57) **Abstract**

A method for inhibiting cell proliferation, cell migration, or tube formation induced by an angiogenic factor. The method includes administrating to a subject in need thereof an effective amount of a compound of the formula: 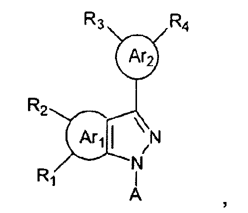 wherein A is H or 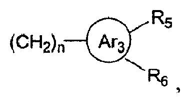 in which n is 0, 1, 2, or 3; each of Ar₁, Ar₂, and Ar₃, independently, is phenyl, thienyl, furyl, pyrrolyl, pyridinyl, or pyrimidinyl; each of R₁, R₂, R₃, R₄, R₅, and R₆, independently, is R, nitro, halogen, C(O)OR, C(O)SR, C(O)NRR', (CH₂)ₘOR, (CH₂)ₘSR, (CH₂)ₘNRR', (CH₂)ₘCN, (CH₂)ₘC(O)OR, (CH₂)ₘCHO, (CH₂)ₘCH=NOR, or R₁ and R₂ together, R₃ and R₄ together, or R₅ and R₆ together are O(CH₂)ₘO, in which each of R and R', independently, is H, alkyl, aryl, heteroaryl, cyclyl, or heterocyclyl; and m is 0, 1, 2, 3, 4, 5, or 6, and n is 0, 1, 2, or 3.

## Description

### RELATED APPLICATIONS

This application is a continuation-in-part of U.S. Application Serial No. 10/147,445, filed on May 16, 2002. It claims priority to both U.S. Application Serial No. 10/147,445 and U.S. Provisional Application No. 60/368,892, filed on March 29, 2002.

### BACKGROUND

When cells in tissues are deprived of oxygen, they release angiogenic factors, e.g., vascular endothelial growth factor (VEGF) and basic fibroblast growth factor (bFGF). The angiogenic factors attract nearby endothelial cells and stimulate them to proliferate, migrate, and form new vessels. This process, known as angiogenesis, occurs in the healthy body for healing wounds and restoring blood flow to tissues after injury. In many disease states, the body loses control over angiogenesis.

Excessive blood vessel growth may be triggered by certain pathological conditions, such as cancer, age-related macular degeneration, rheumatoid arthritis, and psoriasis. As a result of excessive angiogenesis, new blood vessels feed diseased tissues and destroy normal tissues. In cancer, the new vessels allow tumor cells to escape into the circulation and lodge in other organs.

Excessive angiogenesis-related disorders include cancer (both solid and hematologic tumors), cardiovascular diseases (e.g., atherosclerosis), chronic inflammation (e.g., rheutatoid arthritis or Crohn's disease), diabetes (e.g., diabetic retinopathy), psoriasis, endometriosis, and adiposity. See, e.g., Pharmacological Reviews 52: 237-2 68, 2001.

### SUMMARY

This invention is based on a surprising discovery that a group of fused pyrazolyl compounds effectively inhibit cell proliferation, cell migration, and tube formation stimulated by an angiogenic factor.

Thus, an aspect of this invention relates to a method for inhibiting angiogenic factor-induced cell proliferation, angiogenic factor-induced cell migration, or angiogenic factor-induced tube formation. The method includes administrating to a subject in need thereof an effective amount of a compound of the formula: 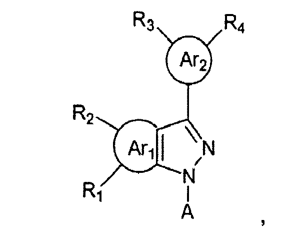
wherein A is H or 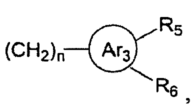 in which n is 0, 1, 2, or 3; each of Ar₁, Ar₂, and Ar₃, independently, is phenyl, thienyl, furyl, pyrrolyl, pyridinyl, or pyrimidinyl; and each of R₁, R₂, R₃, R₄, R₅, and R₆, independently, is R, nitro, halogen, C(O)OR, C(O)SR, C(O)NRR', (CH₂)ₘOR, (CH₂)ₘSR, (CH₂)ₘNRR', (CH₂)ₘCN, (CH₂)ₘC(O)OR, (CH₂)ₘCHO, (CH₂)ₘCH=NOR, or R₁ and R₂ together, R₃ and R₄ together, or R₅ and R₆ together are O(CH₂)ₘO, in which each of R and R', independently, is H, alkyl, aryl, heteroaryl, cyclyl, or heterocyclyl; and m is 0, 1, 2, 3, 4, 5, or 6. Note that when there are one or more R or (CH₂)ₘ moieties in a fused pyrazolyl compound, the R or the (CH₂)ₘ moieties can be the same or different. The angiogenic factor can be VEGF or bFG.F.

Referring to the above formula, a subset of the compounds feature by that Ar₁ is phenyl or thienyl, Ar₂ is 5'-furyl, or Ar₃ is phenyl. Further, when Ar₂ is 5'-furyl, one of R₃ and R₄ can be H, and the other can be CH₂OH. In some embodiment, the CH₂OH group is substituted at position 2 of the furyl.

Shown below are exemplary compounds used to practice the above-described method:

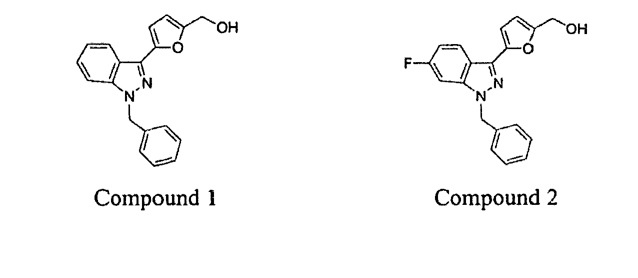

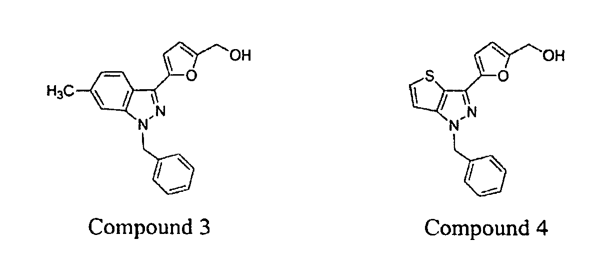

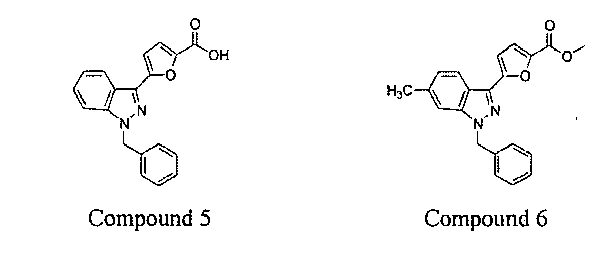

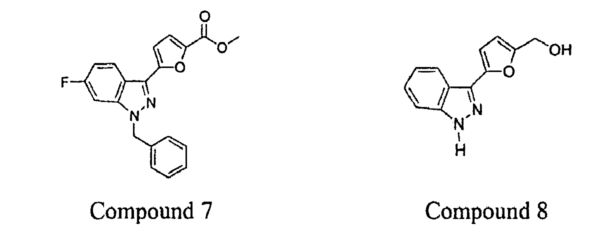

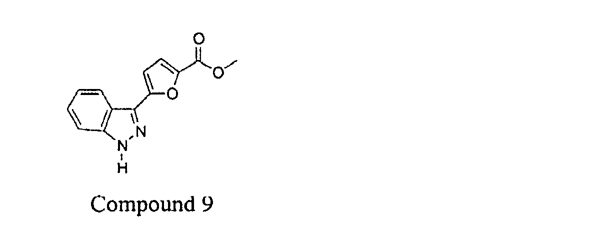

The term "alkyl" refers to a straight or branched hydrocarbon, containing 1-10 carbon atoms. Examples of alkyl groups include, but are not limited to, methyl, ethyl, *n*-propyl, *i-*propyl, *n*-butyl, *i*-butyl, and *t*-butyl.

The term "aryl" refers to a 6-carbon monocyclic, 10-carbon bicyclic, 14-carbon tricyclic aromatic ring system. Examples of aryl groups include, but are not limited to, phenyl, naphthyl, and anthracenyl.

The term "heteroaryl" refers to an aromatic 5-8 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms selected from O, N, or S (e.g., carbon atoms and 1-3, 1-6, or 1-9 heteroatoms of N, O, or S if monocyclic, bicyclic, or tricyclic, respectively). Examples of heteroaryl groups include, but are not limited to, pyridyl, furyl or furanyl, imidazolyl, benzimidazolyl, pyrimidinyl, thiophenyl or thienyl, quinolinyl, indolyl, thiazolyl, and the like.

The term "cyclyl" refers to a saturated and partially unsaturated cyclic hydrocarbon group having 3 to 12 carbons, preferably 3 to 8 carbons, and more preferably 3 to 6 carbons. Examples of cyclyl groups include, but are not limited to, cyclopropyl, cyclobutyl, cyclopentyl, cyclopentenyl, cyclohexyl, cyclohexenyl, cycloheptyl, and cyclooctyl.

The term "heterocyclyl" refers to a nonaromatic 5-8 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms selected from O, N, or S (e.g., carbon atoms and 1-3, 1-6, or 1-9 heteroatoms ofN, O, or S if monocyclic, bicyclic, or tricyclic, respectively). Examples of heterocyclyl groups include piperazinyl, pyrrolidinyl, dioxanyl, morpholinyl, tetrahydrofuranyl, and the like.

Alkyl, aryl, heteroaryl, cyclyl, and heterocyclyl mentioned herein include both substituted and unsubstituted moieties. Examples of substituents include, but are not limited to, halo, hydroxyl, amino, cyano, nitro, mercapto, alkoxycarbonyl, amido, carboxy, alkanesulfonyl, alkylcarbonyl, carbamido, carbamyl, carboxyl, thioureido, thiocyanato, sulfonamido, alkyl, alkenyl, alkynyl, alkyloxy, aryl, heteroaryl, cyclyl, and heterocyclyl, in which the alkyl, alkenyl, alkynyl, alkyloxy, aryl, heteroaryl, cyclyl, and heterocyclyl may be further substituted.

The fused pyrazolyl compound described above can be the compound itself, as well as its salts and prodrugs, if applicable. Such salts, for example, can be formed by interaction between a negatively charged substituent (e.g., carboxylate) on a fused pyrazolyl compound and a cation. Suitable cations include, but are not limited to, sodium ion, potassium ion, magnesium ion, calcium ion, and an ammonium cation such as teteramethylammonium ion. Likewise, a positively charged substituent (e.g., amino) can form a salt with a negatively charged counterion. Suitable counterions include, but are not limited to, chloride, bromide, iodide, sulfate, nitrate, phosphate, or acetate. Examples ofprodrugs include esters and other pharmaceutically acceptable derivatives, which, upon administration to a subject, are capable of providing the fused pyrazolyl compounds described above.

Other features, objects, and advantages of the invention will be apparent from the description, and from the claims.

### DETAILED DESCRIPTION

This invention features a method for inhibiting angiogenic factor-induced cell proliferation, angiogenic factor-induced cell migration, or angiogenic factor-induced tube formation. The method includes administrating to a subject in need thereof an effective amount of a fused pyrazolyl compound. "An effective amount" is defined as the amount of a fused pyrazolyl compound which, upon administration to a subject in need thereof, is required to confer the above-described effect on the subject. The effective amount varies, as recognized by those skilled in the art, depending on the types of the effects, route of administration, excipient usage, and the possibility of co-usage with other treatment.

To practice the method of the present invention, a fused pyrazolyl compound can be administered orally, parenterally, by inhalation spray, or via an implanted reservoir. The term "parenteral" as used herein includes subcutaneous, intracutaneous, intravenous, intramuscular, intraarticular, intraarterial, intrasynovial, intrasternal, intrathecal, intralesional and intracranial injection or infusion techniques.

A composition for oral administration can be any orally acceptable dosage form including, but not limited to, tablets, capsules, emulsions and aqueous suspensions, dispersions and solutions. Commonly used carriers for tablets include lactose and corn starch. Lubricating agents, such as magnesium stearate, are also typically added to tablets. For oral administration in a capsule form, useful diluents include lactose and dried corn starch. When aqueous suspensions or emulsions are administered orally, the active ingredient can be suspended or dissolved in an oily phase combined with emulsifying or suspending agents. If desired, certain sweetening, flavoring, or coloring agents can be added.

A sterile injectable composition (e.g., aqueous or oleaginous suspension) can be formulated according to techniques known in the art using suitable dispersing or wetting agents (such as, for example, Tween 80) and suspending agents. The sterile injectable preparation can also be a sterile injectable solution or suspension in a non-toxic parenterally acceptable diluent or solvent, for example, as a solution in 1,3-butanediol. Among the acceptable vehicles and solvents that can be employed are mannitol, water, Ringer's solution and isotonic sodium chloride solution. In addition, sterile, fixed oils are conventionally employed as a solvent or suspending medium (e.g., synthetic mono- or diglycerides). Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions can also contain a long-chain alcohol diluent or dispersant, or carboxymethyl cellulose or similar dispersing agents.

An inhalation composition can be prepared according to techniques well-known in the art of pharmaceutical formulation and can be prepared as solutions in saline, employing benzyl alcohol or other suitable preservatives, absorption promoters to enhance bioavailability, fluorocarbons, and/or other solubilizing or dispersing agents known in the art.

A carrier in a pharmaceutical composition must be "acceptable" in the sense of being compatible with the active ingredient of the formulation (and preferably, capable of stabilizing it) and not deleterious to the subject to be treated. For example, solubilizing agents, such as cyclodextrins (which form specific, more soluble complexes with fused pyrazolyl compounds), can be utilized as pharmaceutical excipients for delivery of fused pyrazolyl compounds. Examples of other carriers include colloidal silicon dioxide, magnesium stearate, cellulose, sodium lauryl sulfate, and D&C Yellow # 10.

The fused pyrazolyl compound used to practice the method of this invention can be prepared by procedures well known to a skilled person in the art (see, e.g., U.S. Patent No. 5,574,168). They include the following synthetic route: An aryl aryl ketone is first prepared by coupling an arylcarbonyl chloride with another aryl compound. Each aryl compound may be mono- or multi-substituted. The ketone then reacts with an arylalkylhydrazine, the aryl group of which may also be mono- or multi-substituted, to form a hydrazone containing three aryl groups. The hydrazone group is transformed into a fused pyrazolyl core via an alkylene linker, another aryl group is fused at 4-C and 5-C of the pyrazolyl core, and the third aryl group is directly connected to 3-C of the pyrazolyl core. Derivatives of the fused pyrazolyl compound may be obtained by modifying the substituents on any of the aryl groups.

The above-mentioned synthetic route may include additional steps, either before or after the steps described specifically herein, to add or remove suitable protecting groups in order to ultimately allow synthesis of the fused pyrazolyl compound. In addition, various synthetic steps may be performed in an alternate order to give the desired compounds. Synthetic chemistry transformations and protecting group methodologies (protection and deprotection) useful in synthesizing applicable fused pyrazolyl compounds are known in the art and include, for example, those described in R. Larock, *Comprehensive Organic Transformations,* VCH Publishers (1989); T.W. Greene and P.G.M. Wuts, *Protective Groups in Organic Synthesis,* 2d. Ed., John Wiley and Sons (1991); L. Fieser and M. Fieser, *Fieser and Fieser's Reagents for Organic Synthesis,* John Wiley and Sons (1994); and L. Paquette, ed., *Encyclopedia of Reagents for Organic Synthesis,* John Wiley and Sons (1995) and subsequent editions thereof.

A fused pyrazolyl compound thus synthesized can be further purified by a method such as column chromatography, high pressure liquid chromatography, or recrystallization.

A suitable *in vitro* assay can be used to preliminarily evaluate the efficacy of a fused pyrazolyl compound in inhibiting cell proliferation, cell migration, and tube formation induced by an angiogenic factor, e.g., bFGF or VEGF. *In vivo* assays can also be performed according to procedures well known in the art. See, e.g., example 5 below.

Without further elaboration, it is believed that the above description has adequately enabled the,present invention. The following specific embodiments are, therefore, to be construed as merely illustrative, and not limitative of the remainder of the disclosure in any way whatsoever. All of the publications cited herein, including U.S. Application Serial No. 10/147,445 and U.S. Provisional Application No. 60/368,892, are hereby incorporated by reference in their entirety.

### Example 1: Synthesis of 1-benzyl-3-(5'-hydroxymethyl-2'-furyl)indazole (Compound 1)

Calcium borohydride was first prepared by stirring anhydrous calcium chloride (88.8 mg, 0.8 mmole) with sodium borohydride (60 mg, 1.6 mmole) in anhydrous THF (20 mL) for 4 hrs. Then a 30 mL THF solution containing 1-benzyl-3-(5'-methoxycarbonyl-2'-furyl)indazole (88.0 mg, 0.27 mmole) was added dropwise to the calcium borohydride solution at 30±2 °C. The mixture was heated under reflux for 6 hrs, cooled, quenched into crushed ice, placed at a reduced pressure to remove THF, and filtered to obtain a solid product. The solid was extracted with dichloromethane. The extract was concentrated to 50 mL and a solid precipitated after petroleum ether was added. The precipitate was collected and purified by column chromatography (silica gel-benzene) to obtain 70.0 mg of 1-benzyl-3-(5'-hydroxymethyl-2'-furyl)indazole (yield: 87%).
mp: 108-109°C.
MS (%), m/z: 304 (M⁺).
IR (KBr) νₘₐₓ: 3350 cm⁻¹ (-OH).
¹H-NMR (DMSO-d₆, 200 MHz) δ: 4.51 (2H, d, J=5.5 Hz, -CH₂O-) 5.31 (1H, t, J=5.5 Hz, -OH), 5.70 (2H, s, =NCH₂-), 6.48 (1H, d, J=3.4 Hz, H-4'), 6.97 (1H, d, J=3.4 Hz, H-3'), 7.21-7.31 (6H, m, H-5, phenyl), 7.45 (1H, t, J=8.2 Hz, H-6), 7.75 (1H, dd, J=8.2, 1.8 Hz, H-7), 8.12 (1H. dd, J=8.2. 1.0 Hz, C4-H).

### Example 2: Inhibition of cell proliferation

Human umbilical vein endothelial cells (HUVECs) were incubated in the absence of Compound 1 (basal and control) or in the presence of Compound 1 (at various concentrations, i.e., 0.01, 0.03, 0.1, 0.3, and 1 µM). VEGF or bFGF was added (except for basal) to induce DNA synthesis, which was detected based on [³H]thymidine incorporation.

The results show that Compound 1 inhibited VEGF- and bFGF-induced cell proliferation of HUVECs in a concentration-dependent manner. Unexpectedly, Compound 1 exhibited very low IC₅₀ values of 9.0 × 10⁻⁸ M and 1.4 × 10⁻⁷ M, for VEGF and bFGF, respectively.

Other fused pyrazolyl compounds were also tested. All the tested compounds inhibited VEGF-induced cell proliferation of HUVECs. Some are as potent as, or even more potent than, Compound 1.

### Example 3: Inhibition of cell migration

Chemotactic migration of HUVECs was measured with a transwell migration apparatus, following the procedure described in Pan et al., (2003) J. Urol. 69:724-72. Briefly, VEGF or bFGF was diluted to 10 ng/ml with M 199 and 0.1 % bovine serum albumin and added to the lower wells of the transwell chamber of the apparatus. HUVECs (2 × 10⁵ cells in 0.2 ml) were added to the upper wells of the transwell chamber and treated with Compound 1 (3-30 µM) for 1 hr. Filters were positioned above the lower wells. The chamber was incubated for 24 hrs at 37°C under 95% air and 5% CO₂. At the end of the incubation, the filters were removed from the apparatus, and the cells were fixed and stained with hematoxylin. Non-migrating cells on top of the filters were wiped off. The filters were mounted and migrating cells attached to the bottom of the filters were counted in 6 random high-power fields under 400x magnification.

Cell migration was calculated as difference between the number of the migrating cells in the Compound 1-treated and control groups. The results show that Compound 1 (3-30 µM) significantly inhibited, in a dose-dependent manner, VEGF- and bFGF-induced cell migration.

### Example 4: Inhibition of tube formation

HUVECs were cultured onto a chamberslide, which was pre-coated with Matrigel (10 mg/mL). The cells were treated with Compound 1 (10 µM) or without Compound 1 (control). VEGF (10 ng/mL) or bFGF (10 ng/mL) was added to induce tube formation. Photos were taken under 100X magnification. The results show that Compound 1 effectively inhibited VEGF- and bFGF-induced formation of networks of elongated endothelial cells.

### OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the present invention, and without departing from the spirit and scope thereof, can make various changes and modifications of the invention to adapt it to various usages and conditions. For example, a compound structurally analogous to a fused pyrazolyl compound can also be used to practice the present invention. Thus, other embodiments are also within the claims.

## Claims

1. A method for inhibiting cell proliferation induced by an angiogenic factor, comprising administrating to a subject in need thereof an effective amount of a compound of the formula: 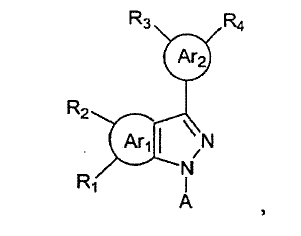
wherein
A is H or 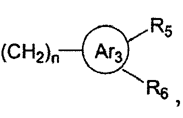 in which n is 0, 1, 2, or 3;
each of Ar₁, Ar₂, and Ar₃, independently, is phenyl, thienyl, furyl, pyrrolyl, pyridinyl, or pyrimidinyl;
each of R₁, R₂, R₃, R₄, R₅, and R₆, independently, is R, nitro, halogen, C(O)OR, C(O)SR, C(O)NRR', (CH₂)ₘOR, (CH₂)ₘSR, (CH₂)ₘNRR', (CH₂)ₘCN, (CH₂)ₘC(O)OR, (CH₂)ₘCHO, (CH2)ₘCH=NOR, or R₁ and R₂ together, R₃ and R₄ together, or R₅ and R₆ together are O(CH₂)ₘO, in which each of R and R', independently, is H, alkyl, aryl, heteroaryl, cyclyl, or heterocyclyl; and m is 0, 1, 2, 3, 4, 5, or 6.

2. The method of claim 1, wherein the angiogenic factor is a vascular endothelial growth factor.

3. The method of claim 1, wherein the angiogenic factor is a basic fibroblast growth factor.

4. The method of claim 1, wherein Ar₃ is phenyl.

5. The method of claim 4, wherein Ar₂ is 5'-furyl.

6. The method of claim 5, wherein R₃ is H and R₄ is CH₂OH and is substituted at position 2 of furyl.

7. The method of claim 6, wherein Ar₁ is phenyl.

8. The method of claim 7, wherein each of R₁ is alkyl or halo, and each of R₂, R₅, and R₆ is H.

9. The method of claim 6, wherein Ar₁ is thienyl.

10. The method of claim 6, wherein the angiogenic factor is a vascular endothelial growth factor or a basic fibroblast growth factor.

11. The method of claim 1, wherein Ar₂ is 5'-furyl.

12. The method of claim 11, wherein R₃ is H and R₄ is CH₂OH and is substituted at position 2 of furyl.

13. The method of claim 12, wherein the angiogenic factor is a vascular endothelial growth factor or a basic fibroblast growth factor.

14. A method for inhibiting cell migration induced by an angiogenic factor, comprising administrating to a subject in need thereof an effective amount of a compound of the formula: 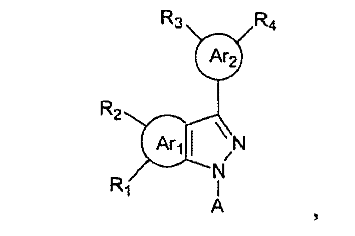
wherein
A is H or 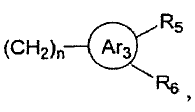
in which n is 0, 1, 2, or 3;
each of Ar₁, Ar₂, and Ar₃, independently, is phenyl, thienyl, furyl, pyrrolyl, pyridinyl, or pyrimidinyl;
each of R₁, R₂, R₃, R₄, R₅, and R₆, independently, is R, nitro, halogen, C(O)OR, C(O)SR, C(O)NRR', (CH₂)ₘOR, (CH₂)ₘSR, (CH₂)ₘNRR', (CH₂)ₘCN, (CH₂)ₘC(O)OR, (CH₂)ₘCHO, (CH₂)ₘCH=NOR, or R₁ and R₂ together, R₃ and R₄ together, or R₅ and R₆ together are O(CH₂)ₘO, in which each of R and R', independently, is H, alkyl, aryl, heteroaryl, cyclyl, or heterocyclyl; and m is 0, 1, 2, 3, 4, 5, or 6.

15. The method of claim 14, wherein the angiogenic factor is a vascular endothelial growth factor.

16. The method of claim 14, wherein the angiogenic factor is a basic fibroblast growth factor.

17. The method of claim 14, wherein Ar₃ is phenyl.

18. The method of claim 17, wherein Ar₂ is 5'-furyl.

19. The method of claim 18, wherein R₃ is H and R₄ is CH₂OH and is substituted at position 2 of furyl.

20. The method of claim 19, wherein Ar₁ is phenyl.

21. The method of claim 20, wherein each of R₁ is alkyl or halo, and each of R₂, R₅, and R₆ is H.

22. The method of claim 19, wherein Ar₁ is thienyl.

23. The method of claim 19, wherein the angiogenic factor is a vascular endothelial growth factor or a basic fibroblast growth factor.

24. The method of claim 14, wherein Ar₂ is 5'-furyl.

25. The method of claim 24, wherein R₃ is H and R₄ is CH₂OH and is substituted at position 2 of furyl.

26. The method of claim 25, wherein the angiogenic factor is a vascular endothelial growth factor or a basic fibroblast growth factor.

27. A method for inhibiting tube formation induced by an angiogenic factor, comprising administrating to a subject in need thereof an effective amount of a compound of the formula: 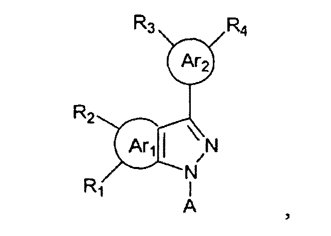
wherein
A is H or 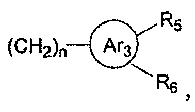
in which n is 0, 1,2, or 3;
each of Ar₁, Ar₂, and Ar₃, independently, is phenyl, thienyl, furyl, pyrrolyl, pyridinyl, or pyrimidinyl; and
each of R₁, R₂, R₃, R₄, R₅, and R₆, independently, is R, nitro, halogen, C(O)OR, C(O)SR, C(O)NRR', (CH₂)ₘOR, (CH₂)ₘSR, (CH₂)ₘNRR', (CH₂)ₘCN, (CH₂)ₘC(O)OR, (CH₂)ₘCHO, (CH₂)ₘCH=NOR, or R₁ and R₂ together, R₃ and R₄ together, or R₅ and R₆ together are O(CH₂)ₘO, in which each of R and R', independently, is H, alkyl, aryl, heteroaryl, cyclyl, or heterocyclyl; and m is 0, 1, 2, 3, 4, 5, or 6.

28. The method of claim 27, wherein the angiogenic factor is a vascular endothelial growth factor.

29. The method of claim 27, wherein the angiogenic factor is a basic fibroblast growth factor.

30. The method of claim 27, wherein Ar₃ is phenyl.

31. The method of claim 30, wherein Ar₂ is 5'-furyl.

32. The method of claim 31, wherein R₃ is H and R₄ is CH₂OH and is substituted at position 2 of furyl.

33. The method of claim 32, wherein Ar₁ is phenyl.

34. The method of claim 33, wherein each of R₁ is alkyl or halo, and each of R₂, R₅, and R₆ is H.

35. The method of claim 32, wherein Ar₁ is thienyl.

36. The method of claim 32, wherein the angiogenic factor is a vascular endothelial growth factor or a basic fibroblast growth factor.

37. The method of claim 27, wherein Ar₂ is 5'-furyl.

38. The method of claim 37, wherein R₃ is H and R₄ is CH₂OH and is substituted at position 2 of furyl.

39. The method of claim 38, wherein the angiogenic factor is a vascular endothelial growth factor or a basic fibroblast growth factor.
